# EUROPÄISCHE PATENTSCHRIFT

(11) **EP 3 363 345 B1**
(45) Veröffentlichungstag und Bekanntmachung des Hinweises auf die Patenterteilung: **30.03.2022**
(21) Anmeldenummer: 18156479.0
(22) Anmeldetag: 13.02.2018
(51) Int. Cl.: A61B 1/00, A61M 25/00, G02B 23/24

(54) **ENDOSKOP, ENDOSKOPKOPF SOWIE VERFAHREN ZUM HERSTELLEN EINES ENDOSKOPS**
ENDOSCOPE, ENDOSCOPE HEAD AND METHOD FOR MANUFACTURING AN ENDOSCOPE
ENDOSCOPE, TÊTE D'ENDOSCOPE AINSI QUE PROCÉDÉ DE FABRICATION D'UN ENDOSCOPE

(30) Priorität: 17.02.2017 DE 102017103267
(43) Veröffentlichungstag der Anmeldung: 22.08.2018
(73) Patentinhaber: Karl Storz SE & Co. KG, 78532 Tuttlingen (DE)
(72) Erfinder: Kärcher, Daniel, 78532 Tuttlingen (DE); Kramer, Claus, 78532 Tuttlingen (DE)

(56) Entgegenhaltungen:
- EP-A1- 1 872 706
- US-A- 4 732 139
- US-A- 5 954 637

## Beschreibung

Die vorliegende Erfindung betrifft ein Endoskop nach dem Oberbegriff von Anspruch 1, einen Endoskopkopf sowie ein Verfahren zum Herstellen eines Endoskops.

Endoskope dienen zur Betrachtung von Hohlräumen im menschlichen oder tierischen Körper sowie in technischen Gegenständen. Ein Endoskop umfasst typischerweise einen lang erstreckten zylindrischen Endoskopschaft, der zur Einführung in den zu betrachtenden Hohlraum geeignet ist, und einen am proximalen (d.h. benutzernahen) Ende des Endoskopschafts angeordneten und fest mit dem proximalen Endbereich des Endoskopschafts verbundenen Endoskopkopf, der Anschlüsse und Bedienelemente sowie einen Okulareinblick aufweisen kann. Innerhalb des Endoskopschafts und des Endoskopkopfs ist ein optisches System zur Weiterleitung eines endoskopischen Bildes vom distalen (d.h. beobachterfernen) Ende des Endoskops zum proximalen Ende angeordnet.

Endoskope sind bei ihrer Benutzung erheblichen mechanischen und thermischen Belastungen ausgesetzt. An die Festigkeit der Verbindung zwischen dem Endoskopkopf und dem Endoskopschaft sind daher hohe Anforderungen gestellt. Dabei müssen sowohl axiale als auch in Querrichtung wirkende Kräfte aufgenommen werden können, und ebenso muss eine Verdrehung des Endoskopschafts gegenüber dem Endoskopkopf um die Längsachse des Endoskopkopfs verhindert werden.

Um den Endoskopschaft auf einfache Weise fest mit dem Endoskopkopf zu verbinden, ist es aus EP 1 872 706 A1 bekannt, den proximalen Endbereich eines Optikrohrs des Endoskopschafts in den distalen Endbereich des Endoskopkopfs einzuschieben und mit diesem durch Stauchen zu verpressen, wobei der distale Endbereich des Endoskopkopfs innenseitig einen umfänglichen Einstich aufweist, in den beim Verpressen Material des Optikrohrs formschlüssig einfließt. Der Einstich wird als vollumfängliche Nut bereitgestellt, so dass besonders viel Material in den Einstich eingreifen kann und die Verbindung des Endoskopkopfs mit dem Optikrohr besonders stabil ausgebildet ist. Um zu verhindern, dass sich das Optikrohr relativ zum Endoskopkopf um seine Längsachse verdrehen kann, wird der distale Endbereich des Endoskopkopfs mit einer umfänglich begrenzten Ausbuchtung bereitgestellt, in die beim Verpressen Material des Optikrohrs formschlüssig einfließt.

Bei dem aus EP 1 872 706 A1 bekannten Endoskop ist die durch Einfließen des Materials in die Ausbuchtung erreichbare Verdrehsicherung nicht immer optimal. Ferner sind zum Einbringen der vollumfänglichen Nut und der Ausbuchtung in den Endoskopkopf mehrere Arbeitsschritte erforderlich, wodurch ein erhöhter Fertigungsaufwand entsteht.

Es ist Aufgabe der vorliegenden Erfindung, ein Endoskop, einen Endoskopkopf sowie ein Verfahren zum Herstellen eines derartigen Endoskops anzugeben, wobei die oben genannten Nachteile möglichst vermieden werden.

Diese Aufgabe wird durch ein Endoskop gemäß Anspruch 1, durch einen Endoskopkopf gemäß Anspruch 11 sowie durch ein Verfahren gemäß Anspruch 12 gelöst.

Vorteilhafte Weiterbildungen der Erfindung ergeben sich aus den Unteransprüchen.

Ein erfindungsgemäßes Endoskop umfasst einen typischerweise langerstreckten Endoskopschaft und einen an einem proximalen Ende des Endoskopschafts angeordneten Endoskopkopf. Der Endoskopschaft weist ein im Wesentlichen zylindrisches Schaftrohr auf, innerhalb dessen beispielsweise ein Objektiv zur Aufnahme eines endoskopischen Bilds und ein Bildweiterleiter zur Weiterleitung des endoskopischen Bilds zum proximalen Ende angeordnet sein können, die in einem innerhalb des Schaftrohrs verlaufenden Innenrohr aufgenommen sein können; es kann aber auch etwa ein elektronischer Bildaufnehmer mit entsprechenden elektrischen Leitungen im Schaftrohr angeordnet sein. Ferner können im Schaftrohr Beleuchtungslichtleiter verlaufen. Das Schaftrohr ist insbesondere ein Außenrohr des Endoskopschafts. Der Endoskopkopf kann eine Okularoptik und eine proximalseitig angeordnete Okularmuschel bzw. einen Anschluss zum Anschließen einer endoskopischen Kamera umfassen. Ein derartiges Endoskop wird auch als Endoskopoptik und der Endoskopkopf als Optikkopf bezeichnet. Das Endoskop ist insbesondere als starres Endoskop mit einem starren Endoskopschaft bzw. einem starren zylindrischen Schaftrohr ausgebildet.

In einem distalen Endbereich weist der Endoskopkopf einen zumindest abschnittsweise zylindrischen Innenraum auf, in den ein proximaler Endbereich des Schaftrohrs eingesetzt ist. Der Endoskopkopf kann hierfür ein Gehäuse mit einem außenseitig beispielsweise zylindrisch oder konisch ausgebildeten distalen Endabschnitt aufweisen, innerhalb dessen der zylindrische Innenraum ausgebildet ist. Der zylindrische Innenraum kann eine Bohrung des Gehäuses sein. Die Achse des zylindrischen Innenraums stimmt im Wesentlichen mit einer Längsachse des Endoskopschafts überein. Die Innenfläche des Endoskopkopfs, die den zylindrischen Innenraum ausbildet, weist eine umlaufende Nut auf. Die Nut kann beispielsweise durch Rotation eines entsprechenden Werkzeugs, das einen Einstich in der Oberfläche des zylindrischen Innenraums erzeugt, ausgeführt werden und kann auch als vollumfänglicher Einstich bezeichnet werden. Das Schaftrohr ist mit seinem proximalen Endbereich in den zylindrischen Innenraum eingesetzt und in die umlaufende Nut eingefaltet. Dies bedeutet, dass Material des Schaftrohrs formschlüssig in die Nut eingreift, vorzugsweise über den gesamten Umfang des Schaftrohrs, so dass das Schaftrohr formschlüssig mit dem Endoskopkopf verbunden ist. Das Schaftrohr kann beispielsweise durch Stauchen in der Längsrichtung mit dem Endoskopkopf verpresst und dabei in die Nut eingefaltet werden.

Erfindungsgemäß ist die umlaufende Nut bezüglich einer Achse des zylindrischen Innenraums, die zumindest näherungsweise einer Längsachse des in diesen eingesetzten zylindrischen Schaftrohrs entspricht, nicht-rotationssymmetrisch ausgebildet. Dies bedeutet insbesondere, dass die Nut umfangsmäßig mit ungleichmäßiger Tiefe und/oder Breite und/oder axialer Position der Nut ausgebildet ist. Somit gibt es mindestens eine erste Umfangsposition, in der die Nut eine erste Tiefe und eine erste Breite hat sowie an einer ersten axialen Position angeordnet ist. Weiter gibt es zumindest eine zweite Umfangsposition der Nut, in der diese eine zweite Tiefe und eine zweite Breite hat und bezüglich der Achse des Innenraums an einer zweiten axialen Position angeordnet ist. Die Nut ist erfindungsgemäß somit insbesondere derart ausgebildet, dass die erste Tiefe ungleich der zweiten Tiefe und/oder die erste Breite ungleich der zweiten Breite und/oder die erste axiale Position ungleich der zweiten axialen Position ist. Die Nut hat vorzugsweise mindestens eine Breite, die dem Doppelten der Wandstärke des Schaftrohrs in dessen proximalem Endbereich entspricht.

Das Schaftrohr und der Innenraum weisen jeweils einen im Wesentlichen kreisförmigen Querschnitt auf, so dass sich ohne eine Verdrehsicherung der Endoskopschaft gegenüber dem Endoskopkopf um die Längsachse verdrehen könnte. Dadurch, dass die umlaufende Nut des Innenraums des Endoskopkopfs nicht-rotationssymmetrisch ausgebildet ist, kann eine Verdrehsicherung des in den Endoskopkopf eingesetzten Schaftrohrs erreicht werden. Insbesondere kann in einem einzigen Arbeitsgang die umlaufende Nut in die innere Oberfläche des Endoskopkopfs, die den zylindrischen Innenraum ausbildet, eingebracht werden; durch Verpressen des Schaftrohrs mit dem Endoskopkopf kann eine feste Verbindung erreicht werden, die zugleich eine Verdrehsicherung gewährleistet. Beim Verpressen etwa durch Stauchen des Schaftrohrs um einen ausreichenden Betrag entsteht eine außenseitige Falte des Schaftrohrs, die formschlüssig in die Nut eingreift und ebenfalls nicht-rotationssymmetrisch ausgebildet ist. Hierdurch kann auf einfache Weise eine besonders wirksame, formschlüssige Verdrehsicherung bewirkt werden.

Vorzugsweise weist die Nut umfangsmäßig eine ungleichmäßige Tiefe auf, d.h. in einer ersten Umfangsposition hat die Nut eine erste Tiefe und in einer zweiten Umfangsposition eine zweite Tiefe, wobei die erste Tiefe größer als die zweite Tiefe ist. Die Tiefe der Nut wird dabei jeweils von einer Innenfläche des Endoskopkopfs, die den zylindrischen Innenraum ausbildet, ausgehend gemessen. Bezogen auf die Achse des zylindrischen Innenraums kann die Nut im Wesentlichen in einer senkrecht zur Achse stehenden Ebene verlaufen. Die Falte, mit der das Schaftrohr in die Nut eingreift, reicht vorzugsweise bis zum Boden der Nut, insbesondere im Wesentlichen in der gesamten umlaufenden Nut oder zumindest in einem Bereich, der über ein Minimum der Tiefe der Nut hinausreicht. In besonders bevorzugter Weise weist die umlaufende Nut zusätzlich eine umfangsmäßig ungleichmäßige Breite auf. Dadurch, dass die Nut eine umfangsmäßig ungleichmäßige Tiefe hat, kann auf besonders einfache und wirksame Weise eine formschlüssige Verdrehsicherung des Schaftrohrs gegenüber dem Endoskopkopf erreicht werden.

Vorzugsweise ist eine maximale Tiefe der Nut, die über den Umfang der Nut erreicht wird, etwa doppelt so groß wie eine minimale Tiefe der Nut. Die maximale Tiefe kann beispielweise etwa 0,4 mm und die minimale Tiefe etwa 0,2 mm betragen. Auf diese Weise kann eine besondere sichere Verdrehsicherung erreicht werden.

In vorteilhafter Weise kann die umlaufende Nut mit einer Mehrzahl von gleichmäßig über den Umfang verteilten Tiefenmaxima und vorzugsweise mittig dazwischenliegenden Tiefenminima ausgebildet sein. Ein Boden der Nut kann somit in einem Querschnitt des Endoskopkopfs eine geschlossene Kurve in Form eines Polygons, vorzugsweise mit abgerundeten Ecken und gekrümmten Seiten, bilden. Die Tiefenmaxima können untereinander jeweils eine gleiche Tiefe aufweisen. Ebenso können die Tiefenminima untereinander jeweils eine gleiche Tiefe haben. Hierdurch wird eine einfache Herstellung ermöglicht und zugleich ein sichere Verbindung des Schaftrohrs mit dem Endoskopkopf, wobei mit erhöhter Sicherheit ein Spiel des Schaftrohrs im Endoskopkopf vermieden werden kann. Alternativ kann es vorgesehen sein, dass die Nut ein einziges Tiefenmaximum und ein diesem vorzugsweise gegenüberliegendes Tiefenminimum aufweist.

In besonders bevorzugter Weise weist die Nut drei näherungsweise im Abstand von 120° entlang des Umfangs angeordnete Tiefenmaxima sowie drei etwa mittig zwischen den Tiefenmaxima angeordnete Tiefenminima auf. Der Boden der Nut stellt somit in einem Querschnitt des Endoskopkopfs eine dreiecksförmige Kurve dar, vorzugsweise mit abgerundeten Ecken und nach außen gekrümmten Seiten. Hierdurch kann besonders sicher ein Spiel des Schaftrohrs gegenüber dem Endoskopkopf vermieden werden. Alternativ können beispielweise jeweils zwei Tiefenmaxima und Tiefenminima oder jeweils vier Tiefenmaxima und Tiefenminima vorgesehen sein.

In weiter vorteilhafter Weise kann die Nut derart ausgebildet sein, dass ein Boden der Nut in einem Querschnitt des Endoskopkopfs, d.h. in einer senkrecht zur Achse des zylindrischen Innenraums stehenden Schnittebene, eine durchgängig nach außen gekrümmte Kurve bildet, der Boden also umlaufend konkav geformt ist. Hierdurch kann einerseits eine besonders einfache Fertigung des Endoskopkopf mit der in die Oberfläche des Innenraums eingebrachten umlaufenden Nut ermöglicht werden und andererseits sichergestellt werden, dass das Schaftrohr im Wesentlichen über den gesamten Umfang in die Nut eingefaltet ist und beispielsweise über den gesamten Umfang bis zum Boden der Nut in diese eingreift.

Gemäß einer bevorzugten Ausführungsform der Erfindung weist die Nut einen trapezförmigen Querschnitt auf. Dabei entspricht ein Querschnitt der Nut, die im Wesentlichen in einer quer zur Achse des zylindrischen Innenraums stehenden Ebene verläuft, einem parallel zur Achse des zylindrischen Innenraums verlaufenden Längsschnitt durch den Endoskopkopf. Eine trapezförmig ausgebildete Nut erlaubt eine besonders einfache Herstellung, beispielsweise mittels eines entsprechend geführten Trapezfräsers, und die Schaffung einer besonders festen Verbindung zwischen dem Schaftrohr und dem Endoskopkopf.

Vorzugsweise beträgt die Schrägstellung der Flanken der trapezförmig ausgebildeten Nut etwa 60° zur Längsachse des zylindrischen Innenraums. Weiter vorzugsweise ist ein Boden der Nut im Längsschnitt parallel zur Achse des zylindrischen Innenraums und damit parallel zur Oberfläche des Innenraums gerichtet. Die Breite des Bodens der Nut kann umfangsmäßig zumindest näherungsweise konstant sein, so dass bei einem konstanten Schrägstellungswinkel der Flanken der trapezförmigen Nut die an der Oberfläche des Innenraums gemessene Breite der Nut umfangsmäßig ungleichmäßig ist, nämlich maximal in Umfangspositionen maximaler Tiefe der Nut und minimal in Umfangspositionen minimaler Tiefe. Hierdurch wird nicht nur eine besonders einfache Fertigung ermöglicht, sondern auch das Einfalten des Schaftrohrs in die Nut beim Stauchen erleichtert.

In weiter bevorzugter Weise ist das Schaftrohr derart in die Nut eingefaltet, dass die Falte des Schaftrohrs, d.h. das in die Nut eingreifende Material des Schaftrohrs, die Nut im Wesentlichen ausfüllt oder zumindest den Boden oder beide Flanken einer trapezförmigen Nut berührt. In besonders bevorzugter Weise füllt die Falte in allen Umfangspositionen die Nut im Wesentlichen aus oder zumindest in Umfangsbereichen, die die Tiefenminima umfassen und über diese hinausreichen. Die Falte, mit der das Schaftrohr in die Nut eingreift, kann je nach Prozessablauf eine gerade bzw. stehende Falte sein oder kann auch schräg zur Achse des Innenraums oder sogar liegend sein. Die Form der Falte kann dabei von der Tiefe der Nut abhängen und umfangsmäßig ungleichmäßig sein. So kann die Falte in Umfangspositionen, wo die Nut tiefer ist, eine andere Form haben als dort, wo die Nut weniger tief ist. Beispielsweise kann die Wand des Schaftrohrs dort, wo die Nut tiefer ist, weniger eng zusammengefaltet sein und die Nut weniger vollständig ausfüllen als dort, wo die Nut eine geringere Tiefe hat. Dadurch, dass die in die Nut eingreifende, durch das Verpressen erzeugte Falte des Schaftrohrs die Nut im Wesentlichen ausfüllt oder zumindest den Boden oder beide Flanken der Nut berührt, kann eine besonders sichere Verdrehsicherung erreicht werden, wobei zugleich ein Spiel des Schaftrohrs relativ zum Endoskopkopf sicher vermieden werden kann.

Gemäß einer bevorzugten Ausführungsform der Erfindung ist in der Innenfläche des Endoskopkopfs, die den zylindrischen Innenraum bildet, eine Längsnut angeordnet, die mit der umlaufenden Nut verbunden ist und über diese in axialer Richtung hinausreicht, wobei die Längsnut im Verbindungsbereich tiefer als die umlaufende Nut ausgebildet ist. Vorzugsweise ist die Längsnut in proximaler Richtung offen und in distaler Richtung geschlossen. Die Längsnut kann auch als Ausbuchtung des Innenraums bezeichnet werden. Die Längsnut, die in die umlaufende Nut mündet und diese übergreift, ermöglicht das Einbringen von Klebstoff, der durch Kapillarkräfte zwischen die Außenfläche des Schaftrohrs und die Innenfläche des Endoskopkopfs gezogen werden kann. Der Klebstoff ermöglicht eine flüssigkeits- und dampfdichte Verbindung des Schaftrohrs mit dem Endoskopkopf und kann zudem eine zusätzliche Verdrehsicherung bewirken. Ferner kann beim Verpressen des Schaftrohrs mit dem Optikkopf Material in die Längsnut eindringen, so dass hierdurch ebenfalls eine zusätzliche Verdrehsicherung bewirkt werden kann.

Ein erfindungsgemäßer Endoskopkopf ist insbesondere als Endoskopkopf für ein starres Endoskop mit einem starren Schaft, der ein starres zylindrisches Schaftrohr aufweist, ausgebildet. Der Endoskopkopf hat ein Gehäuse, das in einem distalen Endbereich einen zylindrischen Innenraum aufweist, der zur Aufnahme eines proximalen Endbereichs des Schaftrohrs ausgebildet ist. Das Gehäuse des Endoskopkopfs umfasst beispielsweise einen zylindrisch oder konisch geformten distalen Endabschnitt, innerhalb dessen der zylindrische Innenraum ausgebildet ist. Die Innenfläche des Endoskopkopfs, die den zylindrischen Innenraum ausbildet, weist eine umlaufende Nut auf. Erfindungsgemäß ist die Nut bezüglich einer Längsachse des zylindrischen Innenraums nicht-rotationssymmetrisch ausgebildet. Hierdurch wird es auf einfache Weise ermöglicht, das Schaftrohr des Endoskops durch Verpressen mit dem distalen Endbereich des Endoskopkopfs formschlüssig mit diesem zu verbinden und dabei zugleich eine Verdrehsicherung zu schaffen.

Insbesondere ist die Nut wie oben beschrieben ausgebildet, beispielsweise in einer quer zur Achse des zylindrischen Innenraums stehenden Ebene mit einer umfangsmäßig ungleichmäßigen Tiefe. Die umlaufende Nut kann etwa einen trapezförmigen Querschnitt mit umfangsmäßig gleichbleibender Neigung der Flanken aufweisen. Zum Einbringen einer solchen Nut, die umfangsmäßig sowohl eine ungleichmäßige Tiefe als auch eine ungleichmäßige Breite hat, in die Oberfläche des zylindrischen Innenraums des Endoskopkopfs kann ein entsprechend ausgestalteter Fräser entlang einer entsprechenden Kurve innerhalb des Innenraums verfahren werden. Zusätzlich kann der Innenraum eine Längsnut aufweisen, die wie oben beschrieben ausgebildet ist und die beispielsweise durch eine Längsfräsung erzeugt werden kann. Der Endoskopkopf ist insbesondere ein Endoskopkopf eines wie zuvor beschrieben ausgebildeten Endoskops.

Gemäß einem erfindungsgemäßen Verfahren zum Herstellen eines Endoskops werden ein im Wesentlichen zylindrisches Schaftrohr und ein Endoskopkopf, der in einem distalen Endbereich einen zylindrischen Innenraum mit einer umlaufenden, nicht-rotationssymmetrischen Nut aufweist, bereitgestellt. Weiter wird ein proximaler Endbereich des Schaftrohrs in den zylindrischen Innenraum des Endoskopkopfs eingeschoben und der proximale Endbereich des Schaftrohrs mit dem distalen Endbereich des Endoskopkopfs verpresst, insbesondere durch Stauchen des Schaftrohrs. Hierdurch wird das Schaftrohr in die umlaufende Nut eingefaltet und formschlüssig mit dem distalen Endbereich des Endoskopkopfs verbunden. Das Herstellungsverfahren kann weitere Schritte umfassen, etwa das Einbringen von optischen und/oder elektronischen Elementen in das Schaftrohr und den Endoskopkopf.

Vorzugsweise wird das Verpressen durch Stauchen des Schaftrohrs ausgeführt. Dabei wird das Schaftrohr um einen solchen Betrag gestaucht, dass Material des Schaftrohrs zumindest abschnittsweise, vorzugsweise über den gesamten Umfang, in die Nut eindringt und dort zumindest beide Flanken oder den Boden berührt. Das Verpressen kann kalt ausgeführt werden.

Weitere Merkmale des Endoskops, des Endoskopkopfs und des Herstellungsverfahrens sind in EP 1 872 706 A1 offenbart, welches Dokument diesbezüglich durch Bezugnahme in die vorliegende Anmeldung aufgenommen wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 ein Ausführungsbeispiel eines erfindungsgemäßen Endoskops in einer Gesamtansicht;
Fig. 2 einen Teil des Gehäuses eines Endoskopkopfs und den proximalen Endbereich des Schaftrohrs des Endoskops gemäß Fig. 1 in einem Längsschnitt;
Fig. 3 einen vergrößerten Ausschnitt aus Fig. 2;
Fig. 4 die umlaufende Nut des Endoskopkopfs in einer schematischen Querschnittsdarstellung;
Fig. 5 einen Teil des Gehäuses des Endoskopkopfs mit eingepresstem Schaftrohr im Längsschnitt;
Fig. 6 einen vergrößerten Ausschnitt aus Fig. 5;
Fig. 7 die umlaufende Nut des Endoskopkopfs mit eingepresstem Schaftrohr in einer schematischen Querschnittsdarstellung.

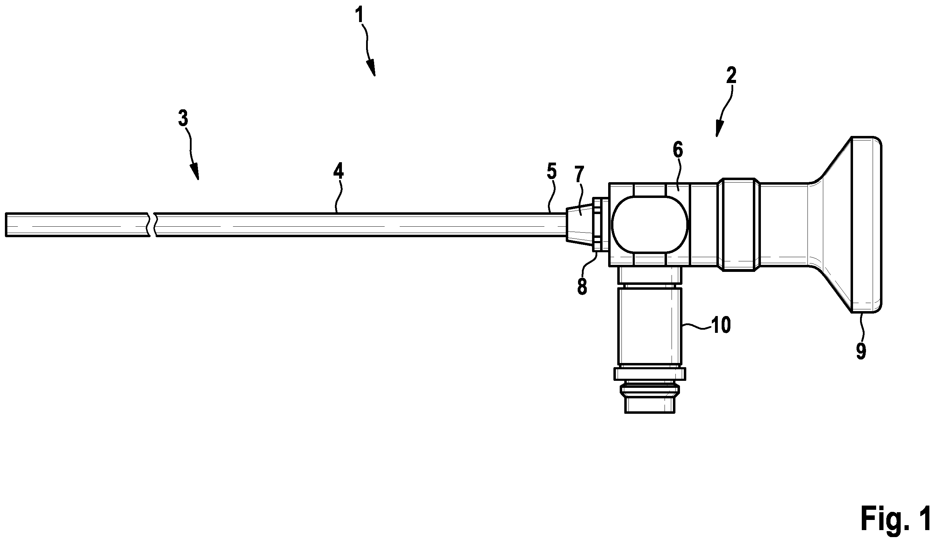

Wie in Fig. 1 exemplarisch dargestellt, umfasst ein erfindungsgemäßes Endoskop 1 einen Endoskopkopf 2 und einen langerstreckten Endoskopschaft 3. Der Endoskopschaft 3 ist bei dem in Fig. 1 gezeigten Endoskop 1, das ein starres Endoskop ist, starr ausgebildet und weist ein Schaftrohr 4 auf, das das Außenrohr des Endoskopschafts 3 bildet. Ein proximaler Endbereich 5 des Schaftrohrs 4 ist fest mit einem Gehäuse 6 des Endoskopkopfs 2 verbunden. Distalseitig weist das Gehäuse 6 eine kegelstumpfförmige Fläche 7 auf, die zum Verbinden mit einem endoskopischen Instrumentenschaft dient, wofür ferner ein Verriegelungselement 8 vorgesehen ist. Proximalseitig trägt das Gehäuse eine Okularmuschel 9. Seitlich am Gehäuse 6 ist ein Lichtanschlussstutzen 10 angeordnet. Innerhalb des Endoskops sind optische Bauelemente aufgenommen, die in den Figuren nicht dargestellt sind. Das Schaftrohr 4 und das Gehäuse 6 des Endoskopkopfs 2 sind beispielsweise aus Edelstahl hergestellt.

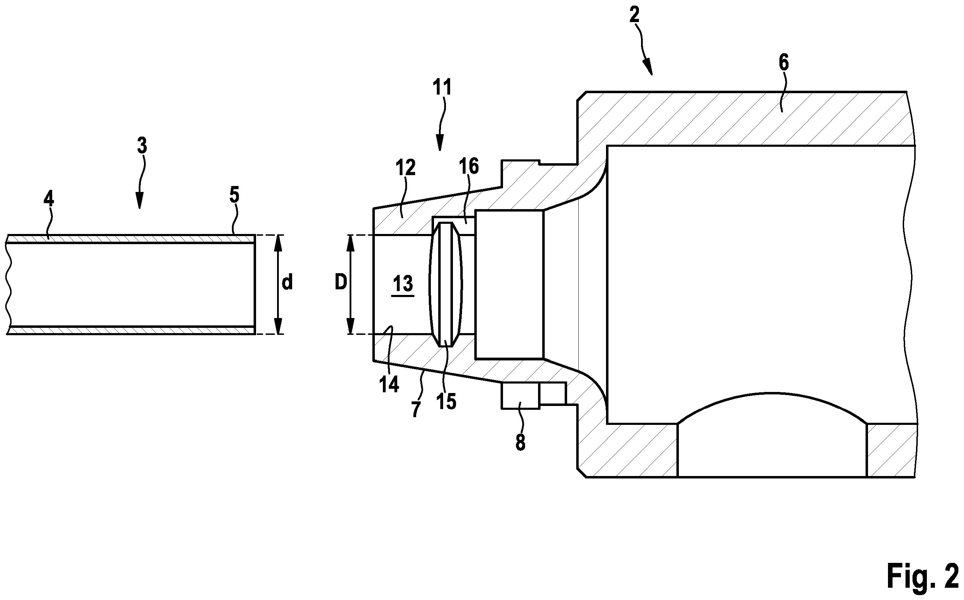

In Fig. 2 ist der distale Bereich des Gehäuses 6 des Endoskopkopfs 2 in einem Längsschnitt gezeigt. Das Gehäuse 6 des Endoskopkopfs 2 weist in seinem distalen Endbereich 11 einen Konus 12 auf, der außenseitig die kegelstumpfförmige Fläche 7 bildet; der Konus 12 kann einstückig mit den übrigen Bereichen des Gehäuses 6 ausgebildet sein. Innenseitig weist der Konus 12 einen zylindrischen Innenraum 13 auf, der sich in axialer Richtung erstreckt und sowohl distalseitig als auch proximalseitig offen ist. Der zylindrische Innenraum 13 kann insbesondere als Durchgangsbohrung des Konus 12 ausgebildet sein. In die Innenfläche 14 des Konus 12 ist eine umlaufende Nut 15 eingebracht, die in einer senkrecht zur Achse des Innenraums 13 stehenden Ebene verläuft. Weiterhin ist in die Innenfläche 14 des Konus 12 eine Längsnut 16 eingebracht, die proximalseitig offen, distalseitig jedoch geschlossen ist und die mit der umlaufenden Nut 15 in Verbindung steht.

In Fig. 2 ist außerdem der proximale Endbereich 5 des Schaftrohrs 4 des Endoskops 1 in einem Längsschnitt dargestellt. Das Schaftrohr 4 ist zumindest im proximalen Endbereich 5 zylindrisch ausgebildet. Der Außendurchmesser d des Schaftrohrs 4 ist nicht größer als der Innendurchmesser D des Konus 12, vorzugsweise geringfügig kleiner als dieser. Der Außendurchmesser d des Schaftrohrs 4 kann beispielsweise 5 mm betragen.

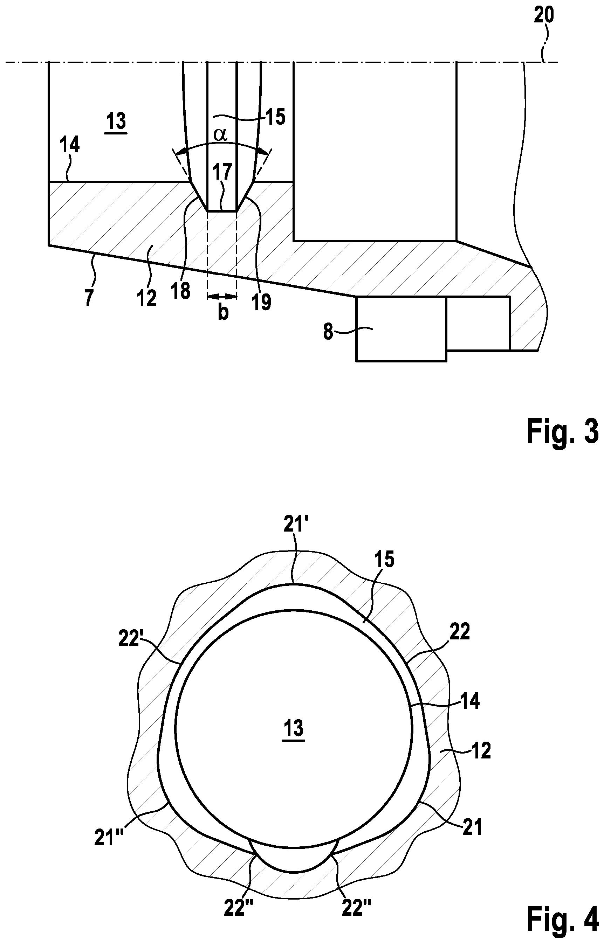

Wie in dem in Fig. 3 gezeigten vergrößerten Ausschnitt des Längsschnitts gemäß Fig. 2 dargestellt ist, weist die umlaufende Nut 15 einen trapezförmigen Querschnitt mit einem Boden 17 und zwei schrägen Flanken 18, 19 auf. Die Flanken 18, 19 stehen zueinander in einem Winkel α = 60°. Der Boden 17 verläuft in dem gezeigten Längsschnitt, der den Querschnitt der Nut 15 zeigt, parallel zur Achse 20 des zylindrischen Innenraums 13. Die Flanken 18, 19 stehen jeweils in einem Winkel von 60° zur Innenfläche 14 des Konus 12 und zum Boden 17 der Nut 15. Im dargestellten Ausführungsbeispiel hat die Nut 15 in der gezeigten Längsschnittebene eine Tiefe von 0,34 mm, und der Boden 16 hat eine Breite b = 0,31 mm.

Die Form der Nut 15 ist in Fig. 4 in einem Querschnitt in der senkrecht zur Achse des Innenraums 13 stehenden Ebene, in der die Nut 15 verläuft, angedeutet. Wie in Fig. 4 dargestellt ist, hat die in die Innenfläche 14 des Konus 12 eingebrachte Nut 15 eine umfangsmäßig ungleichmäßige Tiefe. In dem gezeigten Ausführungsbeispiel weist die Nut 15 drei jeweils um 120° voneinander beabstandete Tiefenmaxima 21, 21', 21" auf, zwischen denen die Tiefenminima 22, 22', 22" angeordnet sind. Der Boden der Nut 15 hat in der Querschnittsdarstellung somit die Form eines abgerundeten Dreiecks. An der Position eines Tiefenminimums 22" ist in die Innenfläche 14 des Konus 12 die Längsnut 16 eingebracht, die mit der umlaufenden Nut 15 verbunden ist und diese untergreift, d.h. im Bereich der Nut 15 tiefer als diese ausgebildet ist. Die Tiefe der Nut 15 beträgt im dargestellten Ausführungsbeispiel in den Tiefenmaxima 21, 21', 21" jeweils etwa 0,4 mm und in den Tiefenminima 22, 22', 22" jeweils etwa 0,2 mm. Im Übrigen ist in Fig. 4 der Konus 12 nicht näher dargestellt.

Zur Montage des Endoskops 1 wird der proximale Endbereich 5 des Schaftrohrs 4 aus distaler axialer Richtung so weit in den Innenraum 13 eingeführt, dass das Schaftrohr 4 proximalseitig ausreichend weit über die Nut 15 hinausragt (s. Fig. 2). Das Schaftrohr 4 wird sodann in den distalen Endbereich 11 des Endoskopkopfs 6 eingepresst. Hierfür wird das Schaftrohr 4 in axialer Richtung gestaucht, wozu es beispielsweise distalseitig in eine Haltevorrichtung eingespannt und aus proximaler Richtung mittels eines eingeführten Dorns gestaucht wird. Hierdurch faltet sich das Schaftrohr 4, das eine entsprechend geringe Wandstärke aufweist, in die Nut 15 ein. Dabei wird das Schaftrohr 4 so weit gestaucht, dass das in die Nut 15 eingedrungene Material die Nut 15 weitgehend ausfüllt oder zumindest außerhalb der Tiefenminima 22, 22', 22" tiefer in die Nut 15 eindringt als der Tiefe der Tiefenminima 22, 22', 22" entspricht. Für ein sicheres Halten des Schaftrohrs 2 im Konus 12 und somit für eine feste Verbindung des Schaftrohrs 2 mit dem Endoskopkopf 6 ist es vorteilhaft, wenn das in die Nut 15 eingedrungene Material des Schaftrohrs 4 im Wesentlichen über den gesamten Umfang zumindest beide Flanken 18, 19 und in besonders vorteilhafter Weise auch den Boden 17 der Nut 15 berührt.

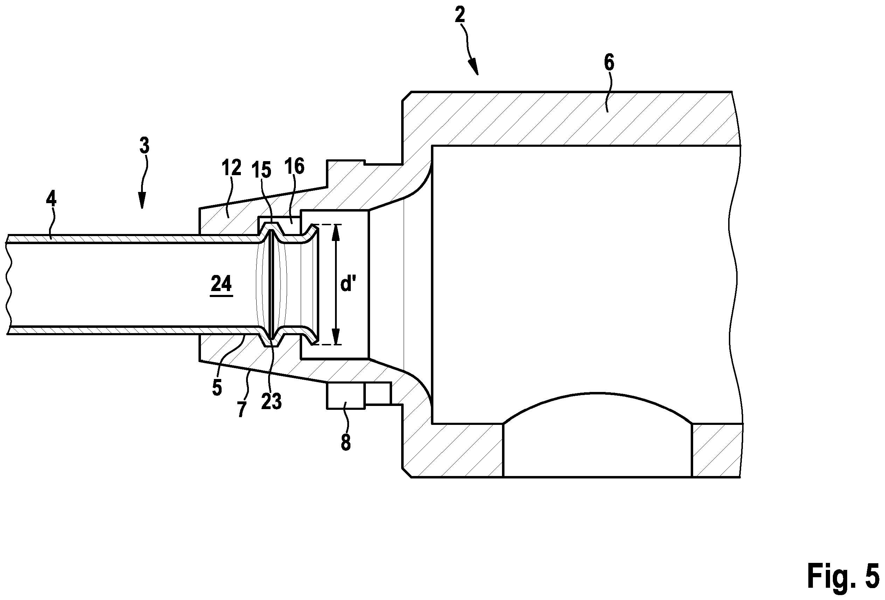

Dies ist in den Figuren 5 bis 7 dargestellt. Wie in dem in Fig. 5 gezeigten Längsschnitt des distalen Bereichs des Gehäuses 6 des Endoskopkopfs 2 gezeigt ist, legt sich beim Stauchen mit einem geeigneten Werkzeug, etwa einem im Wesentlichen zylindrischen Dorn, die Wand des Schaftrohrs 4 als Falte 23 in die Nut 15 ein. Die Falte 23 füllt die Nut 15 teilweise oder vollständig aus und fixiert damit das Schaftrohr 4 im Konus 12; hierdurch wird eine feste Verbindung zwischen dem Schaftrohr 4 und dem Gehäuse 6 des Endoskopkopfs 2 und ein innerhalb des Schaftrohrs 4 bis in den Endoskopkopf 2 durchgehender Hohlraum 24 geschaffen. Insbesondere ist das Schaftrohr 4 hierdurch nicht nur bezüglich axialer und Biegebeanspruchungen fest im Konus 12 gehalten, sondern auch gegen Torsion um die Längsachse relativ zum Gehäuse 6 des Endoskopkopfs 2 fixiert.

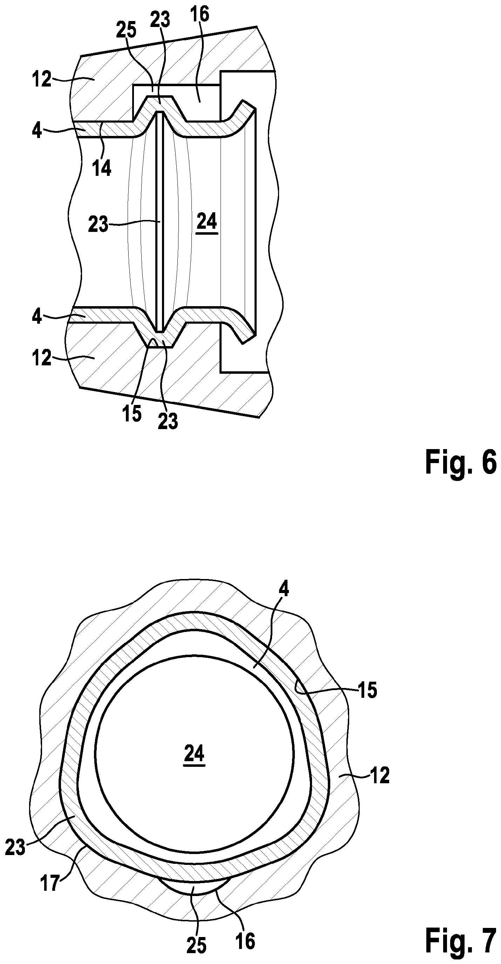

In Fig. 6 ist ein Detail des Längsschnitts gemäß Fig. 5 vergrößert dargestellt. Wie in Fig. 6 zu erkennen ist, legt sich die Falte 23 des Schaftrohrs 4 in die Nut 15 ein und füllt diese weitgehend aus. Die Falte 23 kann auch in die Längsnut 16 eindringen, dies ist für eine Verdrehsicherung jedoch nicht notwendig. Die Längsnut 16 wird nicht bis zum Boden ausgefüllt.

Dies ist auch in dem in Fig. 7 gezeigten Querschnitt, der der Darstellung der Fig. 4 entspricht, gezeigt. Die Falte 23 füllt die Nut 15 über den gesamten Umfang, einschließlich der Tiefenmaxima 21, 21', 21" und der Tiefenminima 22, 22', 22" praktisch bis zum Boden aus. Auch die Längsnut 16 wird teilweise ausgefüllt, jedoch nicht bis zum Boden, so dass ein Zwischenraum 25 verbleibt.

In einem auf das Stauchen folgenden Arbeitsschritt kann proximalseitig Klebstoff in die Längsnut 16 eingebracht werden. Dieser gelangt durch den Zwischenraum 25 zur distalen Seite der Falte 23 (s. Fig. 6). Der Klebstoff dringt sowohl proximal- als auch distalseitig in einen Spalt zwischen der Außenseite des Schaftrohrs 4 und der Innenfläche 14 des Konus 12 ein, härtet darin aus und ermöglicht somit eine flüssigkeits- und dampfdichte Verbindung des Schaftrohrs 4 mit dem Gehäuse 6 des Endoskopkopfs 2.

Ferner kann, wie in Fig. 5 durch den vergrößerten Außendurchmesser d' angedeutet ist, das Schaftrohr 4 durch Einführen eines entsprechenden Doms proximalseitig trichterförmig erweitert werden. Im Übrigen kann das Verpressen des Schaftrohrs 4 mit dem distalen Endbereich des Endoskopkopfs 2 ausgeführt werden wie in EP 1 872 706 A1 beschrieben.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Endoskop
- 2: Endoskopkopf
- 3: Endoskop schaft
- 4: Schaftrohr
- 5: Proximaler Endbereich des Schaftrohrs
- 6: Gehäuse
- 7: Fläche
- 8: Verriegelungselement
- 9: Okularmuschel
- 10: Lichtanschlussstutzen
- 11: Distaler Endbereich des Endoskopkopfs
- 12: Konus
- 13: Innenraum
- 14: Innenfläche
- 15: Nut
- 16: Längsnut
- 17: Boden
- 18: Flanke
- 19: Flanke
- 20: Achse
- 21, 21', 21": Tiefenmaximum
- 22, 22', 22": Tiefenminimum
- 23: Falte
- 24: Hohlraum
- 25: Zwischenraum

## Patentansprüche

1. Endoskop mit einem Endoskopschaft (3), der ein zylindrisches Schaftrohr (4) aufweist, und einem an einem proximalen Ende des Endoskopschafts (3) angeordneten Endoskopkopf (2), der in einem distalen Endbereich (11) einen zylindrischen Innenraum (13) mit einer umlaufenden Nut (15) aufweist, wobei das Schaftrohr (4) in den zylindrischen Innenraum (13) eingesetzt und in die umlaufende Nut (15) eingefaltet ist, wobei ein Boden (17) der Nut (15) eine geschlossene, durchgängig konkav gekrümmte Kurve bildet, **dadurch gekennzeichnet, dass** die Nut (15) nicht-rotationssymmetrisch ausgebildet ist.

2. Endoskop nach Anspruch 1, **dadurch gekennzeichnet, dass** die Nut (15) umfangsmäßig eine ungleichmäßige Tiefe aufweist.

3. Endoskop nach Anspruch 2, **dadurch gekennzeichnet, dass** eine maximale Tiefe der Nut (15) etwa doppelt so groß wie eine minimale Tiefe ist.

4. Endoskop nach Anspruch 2 oder 3, **dadurch gekennzeichnet, dass** die Nut (15) eine Mehrzahl von gleichmäßig über ihren Umfang verteilten Tiefenmaxima (21, 21', 21") und dazwischenliegende Tiefenminima (22, 22', 22") aufweist.

5. Endoskop nach Anspruch 4, **dadurch gekennzeichnet, dass** die Nut (15) drei Tiefenmaxima (21, 21', 21") und drei Tiefenminima (22, 22', 22") aufweist.

6. Endoskop nach einem der vorhergehenden Ansprüche, **dadurch gekennzeichnet, dass** die Nut (15) in einem Querschnitt der Nut (15), einem parallel zur Achse des zylindrischen Innenraums (13) verlaufenden Längsschnitt durch den Endoskopkopf (2) trapezförmig ausgebildet ist.

7. Endoskop nach einem der vorhergehenden Ansprüche, **dadurch gekennzeichnet, dass** die Nut (15) einen Boden (17) und beidseitig angeordnete Flanken (18, 19) aufweist, wobei eine Schrägstellung der Flanken etwa 60° beträgt.

8. Endoskop nach einem der vorhergehenden Ansprüche, **dadurch gekennzeichnet, dass** eine Falte (23) des Schaftrohrs (4) die Nut (15) im Wesentlichen ausfüllt.

9. Endoskop nach einem der vorhergehenden Ansprüche, **dadurch gekennzeichnet, dass** der zylindrische Innenraum (13) eine Längsnut (16) aufweist, die mit der umlaufenden Nut (15) verbunden ist und in axialer Richtung über diese hinausreicht, wobei die Längsnut (16) im Verbindungsbereich tiefer als die umlaufende Nut (15) ausgebildet ist.

10. Endoskopkopf, insbesondere für ein starres Endoskop (1), mit einem Gehäuse (6), das in einem distalen Endbereich (11) einen zylindrischen Innenraum (13) mit einer umlaufenden Nut (15) aufweist, wobei ein Boden (17) der Nut (15) eine geschlossene, durchgängig konkav gekrümmte Kurve bildet, **dadurch gekennzeichnet, dass** die umlaufende Nut (15) nicht-rotationssymmetrisch ausgebildet ist.

11. Verfahren zum Herstellen eines Endoskops, wobei ein zylindrisches Schaftrohr (4) und ein Endoskopkopf (2), der in einem distalen Endbereich (11) einen zylindrischen Innenraum (13) mit einer umlaufenden, nicht-rotationssymmetrischen Nut (15) aufweist, bereitgestellt werden, wobei ein Boden (17) der Nut (15) eine geschlossene, durchgängig konkav gekrümmte Kurve bildet, ein proximaler Endbereich (5) des Schaftrohrs (4) in den zylindrischen Innenraum (13) des Endoskopkopfs (2) eingeschoben wird und der proximale Endbereich (5) des Schaftrohrs (4) mit dem distalen Endbereich (11) des Endoskopkopfs (2) verpresst wird.

## Claims

1. Endoscope with an endoscope shaft (3), which has a cylindrical shaft tube (4), and with an endoscope head (2), which is arranged at a proximal end of the endoscope shaft (3) and, in a distal end region (11), has a cylindrical interior (13) with a peripheral groove (15), wherein the shaft tube (4) is inserted into the cylindrical interior (13) and folded into the peripheral groove (15), wherein a bottom (17) of the groove (15) forms a closed, continuously concave curve, **characterized in that** the groove (15) is not rotationally symmetrical.

2. Endoscope according to Claim 1, **characterized in that** the groove (15) has a non-uniform depth about the circumference.

3. Endoscope according to Claim 2, **characterized in that** a maximum depth of the groove (15) is about twice as great as a minimum depth.

4. Endoscope according to Claim 2 or 3, **characterized in that** the groove (15) has a plurality of depth maxima (21, 21', 21ʺ) distributed uniformly about its circumference and, lying between these, a plurality of depth minima (22, 22', 22ʺ).

5. Endoscope according to Claim 4, **characterized in that** the groove (15) has three depth maxima (21, 21', 21ʺ) and three depth minima (22, 22', 22ʺ).

6. Endoscope according to one of the preceding claims, **characterized in that** the groove (15) has a trapezoid shape in a cross section of the groove (15) to a longitudinal section running through the endoscope head (2) parallel to the axis of the cylindrical interior (13).

7. Endoscope according to one of the preceding claims, **characterized in that** the groove (15) has a bottom (17) and, arranged on both sides, flanks (18, 19), wherein an inclination of the flanks measures approximately 60°.

8. Endoscope according to one of the preceding claims, **characterized in that** a fold (23) of the shaft tube (4) substantially fills the groove (15).

9. Endoscope according to one of the preceding claims, **characterized in that** the cylindrical interior (13) has a longitudinal groove (16) which is connected to the peripheral groove (15) and extends beyond the latter in the axial direction, wherein the longitudinal groove (16) is deeper than the peripheral groove (15) in the connecting region.

10. Endoscope head, in particular for a rigid endoscope (1), with a housing (6) which, in a distal end region (11), has a cylindrical interior (13) with a peripheral groove (15), wherein a bottom (17) of the groove (15) forms a closed, continuously concave curve, **characterized in that** the peripheral groove (15) is not rotationally symmetrical.

11. Method for producing an endoscope, wherein a cylindrical shaft tube (4) and an endoscope head (2) are made available, the latter having, in a distal end region (11), a cylindrical interior (13) with a peripheral, non-rotationally symmetrical groove (15), wherein a bottom (17) of the groove (15) forms a closed, continuously concave curve, a proximal end region (5) of the shaft tube (4) is pushed into the cylindrical interior (13) of the endoscope head (2), and the proximal end region (5) of the shaft tube (4) is press-fitted to the distal end region (11) of the endoscope head (2).

## Revendications

1. Endoscope comprenant une tige d'endoscope (3) qui comporte un tube de tige cylindrique (4) et une tête d'endoscope (2) qui est disposée à une extrémité proximale de la tige d'endoscope (3) et qui comporte dans une zone d'extrémité distale (11) un espace intérieur cylindrique (13) pourvu d'une rainure circonférentielle (15), le tube de tige (4) étant inséré dans l'espace intérieur cylindrique (13) et étant plié dans la rainure circonférentielle (15), un fond (17) de la rainure (15) formant une courbe fermée incurvée continument de manière concave, **caractérisé en ce que** la rainure (15) est conçue pour ne pas être à symétrie de révolution.

2. Endoscope selon la revendication 1, **caractérisé en ce que** la rainure (15) a une profondeur circonférentiellement non uniforme.

3. Endoscope selon la revendication 2, **caractérisé en ce qu'**une profondeur maximale de la rainure (15) est environ deux fois plus grande qu'une profondeur minimale.

4. Endoscope selon la revendication 2 ou 3, **caractérisé en ce que** la rainure (15) comporte une pluralité de maxima de profondeur (21, 21', 21ʺ) et de minima de profondeur intermédiaire (22, 22', 22ʺ) répartis régulièrement sur sa circonférence.

5. Endoscope selon la revendication 4, **caractérisé en ce que** la rainure (15) comporte trois maxima de profondeur (21, 21', 21ʺ) et trois minima de profondeur (22, 22', 22ʺ).

6. Endoscope selon l'une des revendications précédentes, **caractérisé en ce que** la rainure (15) est trapézoïdale dans une section transversale de la rainure (15) à une section longitudinale à travers la tête d'endoscope (2) parallèlement à l'axe de l'espace intérieur cylindrique (13).

7. Endoscope selon l'une des revendications précédentes, **caractérisé en ce que** la rainure (15) comporte un fond (17) et des flancs (18, 19) disposés de part et d'autre, l'inclinaison des flancs étant d'environ 60°.

8. Endoscope selon l'une des revendications précédentes, **caractérisé en ce qu'**un pli (23) du tube de tige (4) remplit sensiblement la rainure (15).

9. Endoscope selon l'une des revendications précédentes, **caractérisé en ce que** l'espace intérieur cylindrique (13) comporte une rainure longitudinale (16) qui est reliée à la rainure circonférentielle (15) et qui s'étend au-delà de celle-ci dans la direction axiale, la rainure longitudinale (16) étant conçue pour être plus profonde dans la zone de liaison que la rainure circonférentielle (15).

10. Tête d'endoscope, destinée notamment à un endoscope rigide (1), ladite tête comprenant un boîtier (6) qui comporte dans une zone d'extrémité distale (11) un espace intérieur cylindrique (13) pourvu d'une rainure circonférentielle (15), un fond (17) de la rainure (15) formant une courbe fermée incurvée continument de manière concave, **caractérisée en ce que** la rainure circonférentielle (15) est conçue pour ne pas être à symétrie de révolution.

11. Procédé de fabrication d'un endoscope, un tube de tige cylindrique (4) et une tête d'endoscope (2) étant produits, laquelle tête comporte dans une zone d'extrémité distale (11) un espace intérieur cylindrique (13) pourvu d'une rainure circonférentielle (15) qui n'est pas à symétrie de révolution, un fond (17) de la rainure (15) formant une courbe fermée incurvée continument de manière concave, une zone d'extrémité proximale (5) du tube de tige (4) étant insérée dans l'espace intérieur cylindrique (13) de la tête d'endoscope (2) et la zone d'extrémité proximale (5) du tube de tige (4) étant pressée avec la zone d'extrémité distale (11) de la tête d'endoscope (2).
